# EUROPÄISCHE PATENTSCHRIFT

(11) **EP 3 024 413 B1**
(45) Veröffentlichungstag und Bekanntmachung des Hinweises auf die Patenterteilung: **05.05.2021**
(21) Anmeldenummer: 13747655.2
(22) Anmeldetag: 24.07.2013
(51) Int. Cl.: A61C 19/045, G16B 5/00

(54) **BESTIMMEN DER LAGE DER KONDYLEN-GELENKACHSE ZUM ERSTELLEN EINES VIRTUELLEN ARTIKULATORS**
DETERMINATION OF THE POSITION OF THE CONDYLAR ARTICULATION AXIS FOR CREATING A VIRTUAL ARTICULATOR
DÉTERMINATION DE LA POSITION DE L'AXE D'ARTICULATION CONDYLIENNE POUR CRÉER UN ARTICULATEUR VIRTUEL

(43) Veröffentlichungstag der Anmeldung: 01.06.2016
(73) Patentinhaber: Sirona Dental Systems GmbH, 64625 Bensheim (DE)
(72) Erfinder: KLEIN, Konrad, 69121 Heidelberg (DE)
(74) Vertreter: Özer, Alpdeniz
(86) Internationale Anmeldenummer: PCT/EP2013/065641
(87) Internationale Veröffentlichungsnummer: WO 2015/010733

(56) Entgegenhaltungen:
- EP-A2- 2 159 724
- WO-A2-2005/115266
- US-A1- 2012 015 316

## Beschreibung

### Gebiet der Erfindung

Die Erfindung betrifft ein Verfahren und eine Vorrichtung zur Erfassung der Kiefergeometrie zur Erstellung eines rechnergestützten, d.h. virtuellen Artikulators. Modelle des Gebisses, und auch Artikulatoren, werden bei der Erstellung dentaler Restaurationen genutzt. Werden diese dentalen Restaurationen rechnergestützt erzeugt (sog. "dentales CAD/CAM"), was beispielsweise bei Restaurationen aus Keramik zwingend ist, ist ein rechnergestütztes Modell erforderlich.

### Stand der Technik

Die Druckschrift EP 2 229 913 A1 beschreibt eine Vorrichtung und ein Verfahren zum Erstellen eines rechnerisch verarbeitbaren Abbilds eines Dentalmodells mit einem Scanner und einer elektronischen Speichereinheit. Nachteilig hieran ist, dass die Möglichkeit der Bewegung des Kiefers nicht berücksichtigt wird, und insbesondere die Kondylen-Gelenkachse nicht erfasst wird und in dem rechnergestützten Modell nicht zur Verfügung steht. Daher können Knirsch-Kau- und Beißbewegungen nicht simuliert werden.

Bekannt ist außerdem die Vorgehensweise, im virtuellen Modell nur die Daten eines konkreten bekannten mechanischen Artikulators vorzusehen. Adapterelemente (z. B. aus Gips), mit denen die realen Ober- und Unterkiefermodelle an den mechanischen Artikulator angepasst werden, müssen zusätzlich eingemessen werden, bevor die Ober- und Unterkiefermodelle im virtuellen Artikulator verwendet werden können, da dieser genau wie der mechanische Artikulator fest vorgegeben ist. Unter anderem ist hierbei nachteilig, dass die Messung verschiedener Komponenten (Modelle des Ober- und Unterkiefers, Adapter für Ober- und Unterkiefer) und ihrer Orientierung zueinander zu erheblichen Ungenauigkeiten im virtuellen Modell führt.

Die Überführung der Maße des mechanischen Artikulators in die digitalisierte Geometrie läuft dabei in mehreren Schritten ab:
1. Einmessen der Orientierung von Adaptergeometrien, an denen Ober- und Unterkiefermodelle im mechanischen Artikulator adaptiert werden, relativ zueinander und zur Kondylen-Gelenkachse des mechanischen Artikulators.
2. Einmessen der Orientierung der artikulatorgleichen Adaptergeometrie, die im 3D-Erfassungssystem montiert ist, relativ zum 3D-Erfassungssystem.
3. Aufnahme von Oberkiefer- und Unterkiefermodell, welches im 3D-Erfassungssystem auf der artikulatorgleichen Adaptergeometrie befestigt wurde.

Aus den beiden Einmess-Vorgängen ergibt sich unmittelbar die Lage von Ober- und Unterkiefermodell zur Artikulator-Geometrie und damit auch zur Kondylen-Gelenkachse. Die beiden Einmess-Vorgänge müssen für jeden mechanischen Artikulator einmalig durchgeführt werden. Dieses Verfahren hat den Nachteil der Fehlerakkumulation über die oben beschriebene Verarbeitungskette:
∘ Das Einmessen des mechanischen Artikulators ist aufwendig und kann beim Anwender nur mit eingeschränkter Genauigkeit erfolgen.
∘ Die Verwendung von Nominalmaßen bringt Fertigungstoleranzen, d.h. Exemplar-spezifische Abweichungen von den Nominalmaßen, in die Verarbeitungskette.
∘ In jedem Fall ist eine fehlerbehaftete, datenabhängige rechnerische Korrektur der Einstellung des Inzisalstiftes am Artikulator nötig, da dieser im Allgemeinen nicht die angenommene Geometrie in ausreichender Genauigkeit reproduziert. Dies ist kritisch, weil für die Korrektur der relativ kleine Kontaktbereich von Oberkiefer zu Unterkiefer (Auflagepunkte) verwendet werden muss. Fehler in den Kontaktbereichen können nicht oder nur wenig durch Mittelung oder Ausreißer-Analyse in ihrem Einfluss eingeschränkt werden.
∘ Das Einmessen der Adaptergeometrie im 3D-Erfassungssystem ist fehlerbehaftet.

### Aufgabe

Aufgabe der Erfindung ist es, ein Verfahren und eine Vorrichtung anzugeben, die den Stand der Technik bei der rechnergestützten Modellierung eines Gebisses verbessern.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Zur Lösung der Aufgabe wird ein Verfahren zum Bestimmen der Lage der Kondylen-Gelenkachse eines Kiefers eines Säugetiers, beispielsweise eines Menschen, relativ zu den Zähnen von Unter- und/oder Oberkiefer vorgeschlagen, welches folgende Schritte umfasst:
- bukkale Aufnahme von Lage und Orientierung der Zähne des Ober- und Unterkiefers in Schlussbissstellung;
- bukkale Aufnahme von Lage und Orientierung der Zähne des Ober- und Unterkiefers in einer Stellung, in der der Unter- oder Oberkiefer gegenüber der Schlussbissstellung um einen Winkel von 1-20°, vorzugsweise 5-10° um die Kondylen-Gelenkachse rotiert ist; und
- rechnerische Bestimmung der Lage der Kondylen-Gelenkachse relativ zu den Zähnen von Unter- und/oder Oberkiefer aus den aufgenommenen Lagen und Orientierungen.

Dadurch wird es möglich, die Lage der Kondylen-Gelenkachse an den Einzelfall angepasst konkret zu bestimmen, ohne über besondere Kenntnisse z. B. der Maße eines bestimmten mechanischen Artikulators oder irgendwelcher Adapterelemente oder deren Anordnung verfügen zu müssen. Die bukkalen Aufnahmen können dabei entweder direkt am Patienten durchgeführt werden, oder auch beispielsweise an einem realen Modell, welches in einen mechanischen Artikulator eingepasst wurde. Als Aufnahmetechniken kommen grundsätzlich alle taktilen oder optischen Messtechniken, oder ggf. auch Röntgen- oder Ultraschalltechniken in Betracht.

Ferner wird zur Lösung der Aufgabe ein Verfahren zum Erstellen eines virtuellen Artikulators für einen Kiefer und das zugehörige Gebiss vorgeschlagen, mit folgenden Schritten:
- Aufnahme eines virtuellen Modells der Zähne des Oberkiefers;
- Aufnahme eines virtuellen Modells der Zähne des Unterkiefers;
- Bestimmen der Lage der Kondylen-Gelenkachse des Kiefers wie oben beschrieben, relativ zu den Zähnen des virtuellen Modells von Unter- und/oder Oberkiefer; und
- Erstellen des virtuellen Artikulators aus den virtuellen Modellen von Unter- und Oberkiefer, ihrer relativen Lage zueinander, sowie der relativen Lage der Kondylen-Gelenkachse.

Mit einem solchen virtuellen Artikulator wird die Herstellung hochwertiger dentaler Restaurationen, z. B. mit Keramikelementen, erheblich verbessert, da diese sowieso nur rechnergestützt hergestellt werden. Ein virtueller Artikulator, der die Kondylen-Gelenkachse berücksichtigt, ermöglicht hierbei auch die Simulation von Kau- und Beißbewegungen, was das konkrete Formen und Anpassen des herzustellenden Zahnersatzes erheblich erleichtert.

Die bei der Beschreibung des Standes der Technik genannten Fehler werden durch das erfindungsgemäße Verfahren vermieden. Die bukkale Registrierung als verbleibende Fehlerquelle hat einen vergleichsweise geringen Einfluss, da hierbei flächenhaft der Abstand zwischen den beteiligten Oberflächenmessungen minimiert wird. Dabei können zudem einzelne Messfehler rechnerisch erkannt und in ihrem Einfluss eingeschränkt werden. Die Lagebestimmung zwischen Ober- und Unterkiefer erfolgt mit der gleichen Genauigkeit wie die Mehrbildintegration innerhalb von Ober- und Unterkiefer.

Als Messverfahren für die Aufnahme der virtuellen Modelle der Zähne kommen grundsätzlich alle taktilen und/oder optischen Messtechniken in Betracht. Vorteilhafterweise werden flächenhaft messende Systeme der optischen Messtechnik eingesetzt, wie sie von Besl [1] und Blais [2] zusammenfassend beschrieben werden. Die Aufnahme eines virtuellen Modells bedeutet hierbei stets, dass mit dem vorliegenden Messverfahren Aufnahmen gemacht werden und ein Computer aus diesen Daten ein virtuelles Modell berechnet. Sollte eine automatische Steuerung des Aufnahmegeräts möglich sein, z. B. mittels eines Roboterarms, kann dieses Verfahren auch vollautomatisch, gesteuert durch den Computer, ablaufen.

Bei einer vorteilhaften Weiterbildung der genannten Verfahren erfolgen die Aufnahmen der Lage und Orientierung der Zähne des Ober- und Unterkiefers und/oder des virtuellen Modells der Zähne des Ober- und Unterkiefers mittels flächiger Triangulation. Dieses Verfahren ist besonders vorteilhaft, da es gesundheitlich unbedenklich, genau und leicht zu handhaben ist.

Des Weiteren wird die Aufgabe gelöst durch eine Vorrichtung zum Bestimmen der Lage der Kondylen-Gelenkachse eines Kiefers relativ zu den Zähnen von Unter- und/oder Oberkiefer mit:
- Aufnahmemitteln zur bukkalen Aufnahme von Lage und Orientierung der Zähne des Ober- und Unterkiefers;
- Mitteln zur rechnerischen Bestimmung der Lage der Kondylen-Gelenkachse relativ zu den Zähnen von Unter- und/oder Oberkiefer aus
- einer mit Hilfe der Aufnahmemittel aufgenommenen bukkalen Aufnahme von Lage und Orientierung der Zähne des Ober- und Unterkiefers in Schlussbissstellung; und
- einer mit Hilfe der Aufnahmemittel aufgenommenen bukkalen Aufnahme von Lage und Orientierung der Zähne des Ober- und Unterkiefers in einer Stellung, in der der Unter- oder Oberkiefer gegenüber der Schlussbissstellung um einen Winkel von 1-20° um die Kondylen-Gelenkachse rotiert ist, also leicht geöffnet ist, wobei Winkel von 5-10° bevorzugt sind.

Eine vorteilhafte Weiterbildung der Erfindung stellt außerdem eine Vorrichtung zum Erstellen eines virtuellen Artikulators für einen Kiefer und das zugehörige Gebiss bereit, mit:
- Mitteln zum dreidimensionalen Aufnehmen der Zähne des Ober- und Unterkiefers;
- Mitteln zum Berechnen eines virtuellen Modells der Zähne des Ober- und Unterkiefers ausgehend von den dreidimensionalen Aufnahmen der Zähne des Ober- und Unterkiefers;
- Mitteln, wie oben beschrieben, zum Bestimmen der Lage der Kondylen-Gelenkachse des Kiefers relativ zu den Zähnen des virtuellen Modells von Unter- und/oder Oberkiefer; und
- Mitteln zum Berechnen des virtuellen Artikulators aus den virtuellen Modellen von Unter- und Oberkiefer, ihrer relativen Lage zueinander, sowie der relativen Lage der Kondylen-Gelenkachse.

Dabei können die Mittel zum dreidimensionalen Aufnehmen der Zähne des Ober- und Unterkiefers und die Aufnahmemittel, die zum Bestimmen der Lage der Kondylen-Gelenkachse dienen, dieselben sein.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Ein Ausführungsbeispiel ist in der Figur schematisch dargestellt. Im Einzelnen zeigt:

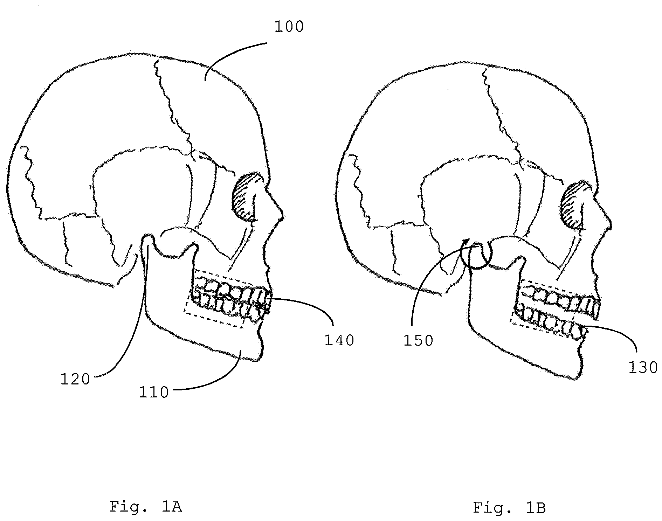

- Fig. 1A: eine Seitenansicht eines menschlichen Schädels in Schlussbissstellung; und
- Fig. 1B: eine Seitenansicht eines menschlichen Schädels mit leicht geöffnetem Kiefer.

Bei einer bevorzugten Ausführungsform des erfindungsgemäßen Verfahrens werden zunächst virtuelle Modelle der Zähne des Ober- und Unterkiefers erfasst. Hierzu wird ein optisches 3D-Messsystem eingesetzt, welches diese Modelle mittels flächiger Triangulation mit Hilfe strukturierter Beleuchtung aufnimmt. Ein Computer berechnet aus diesen Aufnahmen virtuelle Modelle. Anschließend werden mit demselben Aufnahmeverfahren mindestens zwei bukkale Aufnahmen gemacht. Die Fig. 1A und 1B zeigen Seitenansichten des Schädels 100 bei diesen Aufnahmen. Der Unterkiefer 110 ist mittels der Kondylen (Gelenkköpfe) 120 über das Kiefergelenk mit dem restlichen Schädel verbunden und kann (vereinfacht) um dieses Gelenk rotieren. Die genaue Form von Ober- und Unterkiefer spielt bei dieser Betrachtung allerdings keine Rolle; es geht v. a. um die Modellierung des Gebisses, also um Lage und Orientierung der Zähne 130 des Unterkiefers und der Zähne 140 des Oberkiefers.

Zuerst wird eine bukkale Aufnahme in Schlussbissstellung gemacht, wie in der linken Hälfte der Figur dargestellt. Im Anschluss wird eine weitere bukkale Aufnahme bei leicht geöffnetem Kiefer gemacht, wobei die Öffnung durch Rotation des Unterkiefers um die Kondylen-Gelenkachse 150 um einen Winkel von typischerweise 5-10° erreicht wird. Dies ist in der rechten Hälfte der Figur gezeigt. Die aufgenommenen Daten werden mit dem Verfahren von Besl und McKay [3] registriert. Der Computer berechnet aus den registrierten Daten die Lage der Kondylen-Gelenkachse im virtuellen Modell, womit ein virtueller Artikulator zur Verfügung steht, der nicht von den Daten irgendeines vorgegebenen Artikulators abhängt, sondern genau zum zu bearbeitenden Gebiss passt. Die Rechnung basiert dabei vorzugsweise auf einem Quaternionen-Ansatz. Die Umrechnung der aus der Registrierung bekannten Rotations-Matrix in ein Quaternion kann grundsätzlich auf ein Eigenvektor-Problem zurückgeführt werden, bevorzugt kommt aber ein Algorithmus mit zahlreichen Fallunterscheidungen zum Einsatz (ähnlich http://www.cg.info.hiroshima-cu.ac.jp/∼miyazaki/knowledge/teche52.html und Q55 in http://www.cs.princeton.edu/∼gewang/projects/darth/stuff/qu at_faq.html). Die translatorische Komponente der Relativorientierung wird dabei auf eine Verschiebung entlang der Rotationsachse abgebildet.

Es sind zahlreiche Abwandlungen und Weiterbildungen der beschriebenen Ausführungsbeispiele verwirklichbar. So können beispielsweise die unterschiedlichsten (3D-) Mess- und Aufnahmeverfahren zur Anwendung kommen.

Des Weiteren können unterschiedliche, dem Fachmann geläufige Registrierungsverfahren zum Einsatz kommen. Auch kann die Registrierung unterschiedlich verlaufen, etwa:
- Bestimmung der Kondylen-Gelenkachse aus zwei bukkalen Bildern, die abschnittsweise miteinander registriert werden, oder
- Bestimmung der Kondylen-Gelenkachse aus zwei bukkalen Bildern, die abschnittsweise mit Oberkiefer- bzw. Unterkiefer-Daten registriert werden.

Ferner können die Eigenschaften des virtuellen Artikulators in vielfältiger Weise in Bezug auf die gewünschte Verwendung desselben abgewandelt werden, ohne dass dadurch das Wesen der Erfindung verändert würde.

### Glossar

### Artikulator

Gerät zur Simulation der Kiefergelenksbewegung. Dazu werden Gipsmodelle der Zahnbögen des Ober- und Unterkiefers in Okklusion in den Artikulator montiert. Anschließend kann die Bewegung der Kiefer zueinander simuliert werden, was zur Anfertigung von Zahnersatz, Teil- und Totalprothesen oder Schienen unerlässlich ist. (Nach
http://de.wikipedia.org/wiki/Artikulator)
Bukkal (auch buccal):
Backenseitig; (von lat. *"bucca",* Backe).

### Gebiss

Als Gebiss bezeichnet man die Gesamtheit der Zähne eines Wirbeltieres. Hier beginnt die Kette der Verdauung: Zahnreihen im Ober- und im Unterkiefer (Schneide-, Eck- und Backenzähne) zerdrücken, zerreißen und zerkleinern die Nahrung. (Nach http://de.wikipedia.org/wiki/Gebiss)

### Kiefer

Der Kiefer ist der Teil des Gesichtsschädels, der bei den meisten Wirbeltieren zur Nahrungsaufnahme dient und deshalb meist bezahnt ist. Er besteht aus dem Oberkiefer (lat. Maxilla) und dem Unterkiefer (lat. Mandibula). Die Zähne sind im Kiefer in den Zahnfächern (Alveolen) über eine Gomphosis (Einkeilung) verankert. Bei den Säugetieren ist der Unterkiefer im Kiefergelenk (Articulatio temporomandibularis) beweglich am Schläfenbein (Os temporale) befestigt. Ober- und Unterkiefer sind also nur indirekt miteinander verbunden. Der Oberkiefer ist bei Säugetieren unbeweglich, bei ihnen wird lediglich der Unterkiefer durch die Kaumuskulatur bewegt.

### (Nach http://de.wikipedia.org/wiki/Kiefer_(Anatomie))

### Kondylen-Gelenkachse des Kiefergelenks

Der Unterkieferknochen besteht aus dem hufeisenförmigen Unterkieferkörper (Corpus mandibulae), von dem jeweils auf beiden Seiten der aufsteigende Ast (Ramus mandibulae) ausgeht. Vom aufsteigenden Ast gehen zwei weitere Fortsätze aus: Der Gelenkfortsatz (Processus condylaris mandibulae bzw. Processus articularis mandibulae) mit seinem walzenförmigen Gelenkkopf (Caput mandibulae oder Condylus) bildet den beweglichen Teil des Kiefergelenks. Die Achse durch die beiden Gelenkköpfe (Kondylen) wird als Kondylen-Gelenkachse bezeichnet. Beim Auf- und Zuklappen rotiert der Unterkiefer gegenüber dem restlichen Schädel um diese Achse, sofern die Winkel klein bleiben.

### Schlussbissstellung

Schlussbissstellung bezeichnet die maximale Interkuspidation (lateinisch cuspis ,der Höcker'). Das ist die Unterkieferhaltung, bei der maximaler Vielpunktkontakt zwischen Unterkiefer- und Oberkieferzähnen besteht.

### Triangulation

Geometrische Methode der optischen Abstandsmessung durch genaue Winkelmessung innerhalb von Dreiecken. Bei Kenntnis der Strahlrichtung und des Abstandes zwischen einer Kamera und einer Lichtquelle kann damit der Abstand von Oberflächenpunkten eines Objekts zur Kamera bestimmt werden. Die Verbindung Kamera - Lichtquelle sowie die beiden Strahlen von und zum Objekt bilden hierbei ein Dreieck. Mittels dieser Methode kann die dreidimensionale Erfassung (Vermessung) der kompletten Oberfläche eines Objektes realisiert werden. Bei der flächigen Triangulation wird das zu vermessende Objekt durch die Lichtquelle sukzessive mit Streifenmustern mit Streifen unterschiedlicher Breite beleuchtet. Daraus lässt sich die Oberfläche des Objekts rechnerisch rekonstruieren. Näheres findet sich dazu auf http://www.uni-stuttgart.de/ito/forschung/forschung_3d/Streifenprojektion/ sowie http://www.uni-stuttgart.de/ito/forschung/forschung_3d/DSFP/.

### zitierte Literatur

### zitierte Patentliteratur

EP 2 229 913 A1
US2012015316

### zitierte Nicht-Patentliteratur

[1] P.J. Besl: "Active Optical Range Imaging Sensors". In J.L.C. Sanz (editor): "Advances in Machine Vision", S. 1-63. Springer-Verlag, New York, 1989.
[2] Francois Blais: "Review of 20 years of range sensor development". Journal of Electronic Imaging, 13(1): 231-240, Jan. 2004.
[3] P.J. Besl & N.D. McKay: "A Method for Registration of 3-D Shapes". IEEE Transaction on Pattern Analysis and Machine Intelligence, Vol. 14, No. 2, Feb. 1992.
   http://www.cg.info.hiroshima-cu.ac.jp/∼miyazaki/knowledge/teche52.html, zuletzt aufgerufen am 02.07.2013 Q55 in
   http://www.cs.princeton.edu/∼gewang/projects/darth/stuff/qu at_faq.html, zuletzt aufgerufen am 02.07.2013

## Patentansprüche

1. Verfahren zum Bestimmen der Lage der Kondylen-Gelenkachse (150) eines Kiefers relativ zu den Zähnen (130, 140) von Unter- und/oder Oberkiefer mit folgenden Schritten:
a) bukkale Aufnahme von Lage und Orientierung der Zähne des Ober- und Unterkiefers in Schlussbissstellung;
b) bukkale Aufnahme von Lage und Orientierung der Zähne des Ober- und Unterkiefers in einer Stellung, in der der Unter- oder Oberkiefer gegenüber der Schlussbissstellung um einen Winkel von 1-20° um die Kondylen-Gelenkachse rotiert ist; und
c) rechnerische Bestimmung der Lage der Kondylen-Gelenkachse relativ zu den Zähnen von Unter- und/oder Oberkiefer aus den aufgenommenen Lagen und Orientierungen.

2. Verfahren zum Erstellen eines virtuellen Artikulators für einen Kiefer und das zugehörige Gebiss, mit folgenden Schritten:
a) Aufnahme eines virtuellen Modells der Zähne (140) des Oberkiefers;
b) Aufnahme eines virtuellen Modells der Zähne (130) des Unterkiefers;
c) Bestimmen der Lage der Kondylen-Gelenkachse (150) des Kiefers nach Anspruch 1 relativ zu den Zähnen des virtuellen Modells von Unter- und/oder Oberkiefer; und
d) Erstellen des virtuellen Artikulators aus den virtuellen Modellen von Unter- und Oberkiefer, ihrer relativen Lage zueinander, sowie der relativen Lage der Kondylen-Gelenkachse (150).

3. Verfahren nach einem der vorhergehenden Ansprüche, **dadurch gekennzeichnet, dass** die Aufnahmen der Lage und Orientierung der Zähne des Ober- und Unterkiefers und/oder des virtuellen Modells der Zähne des Ober- und Unterkiefers mittels flächiger Triangulation erfolgt.

4. Vorrichtung zum Bestimmen der Lage der Kondylen-Gelenkachse eines Kiefers relativ zu den Zähnen von Unter- und/oder Oberkiefer mit:
a) Aufnahmemitteln zur bukkalen Aufnahme von Lage und Orientierung der Zähne des Ober- und Unterkiefers;
b) Mitteln zur rechnerischen Bestimmung der Lage der Kondylen-Gelenkachse relativ zu den Zähnen von Unter- und/oder Oberkiefer aus
b1) einer mit Hilfe der Aufnahmemittel aufgenommenen bukkalen Aufnahme von Lage und Orientierung der Zähne des Ober- und Unterkiefers in Schlussbissstellung; und
b2) einer mit Hilfe der Aufnahmemittel aufgenommenen bukkalen Aufnahme von Lage und Orientierung der Zähne des Ober- und Unterkiefers in einer Stellung, in der der Unter- oder Oberkiefer gegenüber der Schlussbissstellung um einen Winkel von 1-20° um die Kondylen-Gelenkachse rotiert ist.

5. Vorrichtung zum Erstellen eines virtuellen Artikulators für einen Kiefer und das zugehörige Gebiss, mit:
a) Mitteln zum dreidimensionalen Aufnehmen der Zähne des Ober- und Unterkiefers;
b) Mitteln zum Berechnen eines virtuellen Modells der Zähne des Ober- und Unterkiefers ausgehend von den dreidimensionalen Aufnahmen der Zähne des Ober- und Unterkiefers;
c) Mitteln gemäß Anspruch 4 zum Bestimmen der Lage der Kondylen-Gelenkachse des Kiefers relativ zu den Zähnen des virtuellen Modells von Unter- und/oder Oberkiefer; und
d) Mitteln zum Berechnen des virtuellen Artikulators aus den virtuellen Modellen von Unter- und Oberkiefer, ihrer relativen Lage zueinander, sowie der relativen Lage der Kondylen-Gelenkachse.

## Claims

1. A method for determining the position of the condylar articulation axis (150) of a jaw relative to the teeth (130, 140) of the lower and/or upper jaw, comprising the following steps:
a) buccally recording the position and orientation of the teeth of the upper and lower jaws in the closed bite position;
b) buccally recording the position and orientation of the teeth of the upper and lower jaws in a position in which the lower or upper jaw is rotated about the condylar articulation axis relative to the closed bite position by an angle of 1-20°; and
c) computationally determining the position of the condylar articulation axis relative to the teeth of the lower and/or upper jaw from the recorded positions and orientations.

2. A method for creating a virtual articulator for a jaw and the associated dentition, comprising the following steps:
a) recording a virtual model of the teeth (140) of the upper jaw;
b) recording a virtual model of the teeth (130) of the lower jaw;
c) determining the position of the condylar articulation axis (150) of the jaw according to claim 1 relative to the teeth of the virtual model of the lower and/or upper jaw; and
d) creating the virtual articulator from the virtual models of the lower and upper jaws, their relative positions to one other, and the relative position of the condylar articulation axis (150).

3. The method according to any one of the preceding claims, **characterised in that** the recording of the position and orientation of the teeth of the upper and lower jaws and/or of the virtual model of the teeth of the upper and lower jaws is by means of surface triangulation.

4. A device for determining the position of the condylar articulation axis of a jaw relative to the teeth of the lower and/or upper jaw, comprising:
a) recording means for the buccal recording of the position and orientation of the teeth of the upper and lower jaws;
b) means for computationally determining the position of the condylar articulation axis relative to the teeth of the lower and/or upper jaws from
b1) a buccal recording, recorded by means of the recording means, of the position and orientation of the teeth of the upper and lower jaws in the closed bite position; and
b2) a buccal recording, recorded by means of the recording means, of the position and orientation of the teeth of the upper and lower jaws in a position in which the lower or upper jaw is rotated about the condylar articulation axis relative to the closed bite position by an angle of 1-20°.

5. A device for creating a virtual articulator for a jaw and the associated dentition, comprising:
a) means for three-dimensionally recording the teeth of the upper and lower jaws;
b) means for calculating a virtual model of the teeth of the upper and lower jaws on the basis of the three-dimensional recordings of the teeth of the upper and lower jaws;
c) means according to claim 4 for determining the position of the condylar articulation axis of the jaw relative to the teeth of the virtual model of the lower and/or upper jaws; and
d) means for calculating the virtual articulator from the virtual models of the lower and upper jaws, their relative position to one another, and the relative position of the condylar articulation axis.

## Revendications

1. Procédé de détermination de la position de l'axe d'articulation condylienne (150) d'une mâchoire par rapport aux dents (130, 140) de la mâchoire inférieure et/ou supérieure comprenant les étapes suivantes :
a) enregistrement buccal de la position et de l'orientation des dents de la mâchoire supérieure et inférieure en position d'intercuspidation maximale ;
b) enregistrement buccal de la position et de l'orientation des dents de la mâchoire supérieure et inférieure dans une position dans laquelle la mâchoire inférieure ou supérieure est tournée selon un angle de 1 à 20° autour de l'axe de l'articulation condylienne par rapport à la position d'intercuspidation maximale ; et
c) détermination par calcul de la position de l'axe de l'articulation condylienne par rapport aux dents de la mâchoire inférieure et/ou supérieure à partir des positions et orientations enregistrées.

2. Procédé pour créer un articulateur virtuel pour une mâchoire et la dentition correspondante, comprenant les étapes suivantes :
a) enregistrement d'un modèle virtuel des dents (140) de la mâchoire supérieure ;
b) enregistrement d'un modèle virtuel des dents (130) de la mâchoire inférieure ;
c) détermination de la position de l'axe d'articulation condylienne (150) de la mâchoire selon la revendication 1 par rapport aux dents du modèle virtuel de la mâchoire inférieure et/ou supérieure ; et
d) création de l'articulateur virtuel à partir des modèles virtuels de la mâchoire inférieure et supérieure, de leur position relative l'une par rapport à l'autre et de la position relative de l'axe de l'articulation condylienne (150).

3. Procédé selon l'une quelconque des revendications précédentes, **caractérisé en ce que** l'enregistrement de la position et de l'orientation des dents de la mâchoire supérieure et inférieure et/ou du modèle virtuel des dents de la mâchoire supérieure et inférieure s'effectue au moyen d'une triangulation plane.

4. Dispositif pour déterminer la position de l'axe de l'articulation condylienne d'une mâchoire par rapport aux dents de la mâchoire inférieure et/ou supérieure comprenant :
a) des moyens d'enregistrement pour l'enregistrement buccal de la position et de l'orientation des dents de la mâchoire supérieure et inférieure ;
b) des moyens pour la détermination par calcul de la position de l'axe de l'articulation condylienne par rapport aux dents de la mâchoire inférieure et/ou supérieure
b1) à partir d'un enregistrement buccal de la position et de l'orientation des dents de la mâchoire supérieure et inférieure dans la position d'intercuspidation maximale, effectué à l'aide des moyens d'enregistrement ; et
b2) à partir d'un enregistrement buccal de la position et de l'orientation des dents de la mâchoire supérieure et inférieure dans une position dans laquelle la mâchoire inférieure ou supérieure est tournée selon un angle de 1 à 20° autour de l'axe de l'articulation condylienne par rapport à la position d'intercuspidation maximale, effectué à l'aide du moyen d'enregistrement.

5. Dispositif pour créer un articulateur virtuel pour une mâchoire et la dentition associée, comprenant :
a) des moyens d'enregistrement tridimensionnel des dents de la mâchoire supérieure et inférieure ;
b) des moyens pour calculer un modèle virtuel des dents de la mâchoire supérieure et inférieure à partir des enregistrements tridimensionnels des dents de la mâchoire supérieure et inférieure ;
c) des moyens selon la revendication 4 pour déterminer la position de l'axe d'articulation condylienne de la mâchoire par rapport aux dents du modèle virtuel de la mâchoire inférieure et/ou supérieure ; et
d) des moyens pour calculer l'articulateur virtuel à partir des modèles virtuels des mâchoires inférieure et supérieure, leur position relative l'une par rapport à l'autre ainsi que la position relative de l'axe de l'articulation condylienne.
